# EUROPEAN PATENT APPLICATION

(11) **EP 4 667 556 A1**
(43) Date of publication of application: **24.12.2025**
(21) Application number: 24183769.9
(22) Date of filing: 21.06.2024
(51) Int. Cl.: C12M 1/12, C12M 1/00, C12M 1/34, C12M 1/36

(54) **BIOREACTOR FOR CULTURING ADHERENT CELLS AND METHOD FOR CULTURING ADHERENT CELLS AND PRODUCING BIOMOLECULES**

(71) Applicant: Exo-Harvest, 6041 Charleroi (BE)
(72) Inventor: PRIEELS, Jean-Paul, 1380 Lasne (BE); SERGEANT, David, 6041 Charleroi (BE)
(74) Representative: Calysta NV

(57) **Abstract**

A bioreactor for growing adherent cells comprising a 3D scaffold and inlet and outlet sensors and a continuous control, an assembly of such bioreactors, and culture processes based on these reactors.

## Description

### Technical field

The present invention relates to a bioreactor system for culturing adherent cells and possibly harvesting cell material.

The present invention also relates to a process to grow adherent cells, comprising to seed the adherent cells in the bioreactor system according to the present invention.

The present invention also relates to a device for high-throughput screening comprising a plurality of bioreactor systems for culturing adherent cells according to the present invention.

### Prior art

In biopharmaceutical industry, large-scale culture of cells at industrial levels is performed for purposes of the production of biomolecules such as for example hormones, enzymes, antibodies, viruses for vaccine, therapeutic proteins, and cell therapies.

The markets of cell therapy, gene therapy, and/or acellular therapy for example with the use of bio-based materials such as extracellular vesicles, are growing rapidly, with promising treatments moving into clinical trials and quickly toward commercialization. However, one therapeutic dose can require huge amounts of cells and even much more of cell products. Therefore, being able to provide a large quantity of cell products in a short amount of time and under the highest manufacturing standards is critical for clinical and commercial successes.

Some cell productions of biomolecules must be performed by anchorage dependent cells, wherein the cells need a surface to adhere to for growth.

The culture of adherent cells on a two-dimensional (2D) surface is known for many years. However, these cultures of adherent cells in 2D surface are limited in terms of cellular density required for large scale production of cells or biomolecules needed in clinical practices and in final product.

More recently, the culture of cells on fixed-bed (microcarrier, microcarrier or in hollow fiber bioreactors allows large three-dimensional (3D) culture of cells. However, these bioreactors are offering limited operational range of physicochemical conditions for the different manufacturing process phases or are not compatible with sensitive cell lines due to high level of shear-stress or exposure to shocks. These bioreactors are not optimal for large-scale production.

A packed-bed bioreactor system is also known wherein a high-cell density is achieved by the culture of cells on a surface, wherein the cell culture medium is perfused along the surface or through a (semi-porous) substrate to provide oxygen and nutrients needed for the cell growth. The cell culture on the surface or on the semi-porous substrate in these packed-bed bioreactor systems is generally non-uniform, resulting in a reduced yield of cell or biomolecule culture or resulting in a cell culture of reduced quality and/or non-constant quality. In addition, the harvesting of a high yield of viable cells in such packed-bed bioreactor is quite difficult. The operational range of this bioreactor technology is limited and, by design, does not allow integration of sensors downstream of the packed-bed. The inventors do consider that such design reduces the optimization of culture conditions and process steps.

To circumvent these problems, EP4363547 describes a fixed-bed bioreactor system for culturing cells, wherein the system includes a cell culture vessel including at least one interior reservoir, an inlet fluidly connected to the reservoir, and an outlet fluidly connected to the reservoir, a fluid flow path to supply fluid to the inlet and receive fluid from the outlet, a media conditioning vessel fluidly connected to the cell culture vessel, an outlet sensor arranged at the outlet of the cell culture vessel. The outlet sensor can detect a parameter of cell culture media exiting the cell culture vessel via the outlet, and the bioreactor system can adjust a parameter of the cell culture media based on the parameter detected by the outlet sensor. Although this document extols the merits of its technology, in practice, the inventors consider there is still a need to dispose of a bioreactor system for adherent cell culture allowing for a precise control in continuous (in real-time) of an homogeneous cell culture inside the scaffold, while allowing for a large-scale (industrial scale) production of cells and/or biomolecules of high quality and constant quality while being simple to operate and affordable in term of costs.

### Objectives of the invention

The present invention aims to overcome the drawbacks of the state of the art, in particular those described above.

In particular, the present invention proposes to provide a bioreactor system for culturing adherent cells allowing for a precise control in continue (in real-time) of a homogeneous cell culture inside the scaffold, while allowing for a large-scale (industrial scale) production of cells and/or biomolecules of high quality and constant quality.

The present invention also proposes a bioreactor which is simple to operate and affordable in term of costs, which is needed to develop a daily clinical practice.

### Summary of the invention

To achieve the aforementioned objectives, the present invention provides a bioreactor system for culturing adherent cells, the system comprising:
- a cell culture vessel comprising at least one interior reservoir, wherein the reservoir comprises a porous scaffold arranged to support the culture of said adherent cells,
- an inlet fluidly connected to the reservoir, and an outlet fluidly connected to the reservoir, wherein the outlet is fluidly connected to the inlet via a recirculation loop of cell culture media, wherein the inlet is fluidly connected to a source of cell culture media,
- at least one inlet sensor arranged at the inlet of the cell culture vessel, wherein the inlet sensor is configured to measure a parameter of cell culture media entering the cell culture vessel via the inlet,
- at least one outlet sensor arranged at the outlet of the cell culture vessel, wherein the outlet sensor is configured to measure a parameter of cell culture media exiting the cell culture vessel via the outlet,
- a pump, able to generate a flux of the cell culture media into the recirculation loop,
- a control element operatively connected to the inlet sensor, the outlet sensor, the pump, and to an actionable element able to continuously modify said parameter of cell culture media, wherein the control element is arranged to determine the difference between the parameter of cell culture media measured at the outlet and at the inlet, and, based on such combined information, continuously adapt said parameter of cell culture media through a control of said actionable element, wherein the inlet and outlet sensors comprise at least an oxygen sensor and wherein the porous scaffold is a 3D printed scaffold.

Indeed, the bioreactor system for culturing adherent cells according to the present invention combining a 3D printed porous scaffold, an inlet and an outlet sensor, and a control element arranged in a recirculation loop to measure continuously the parameter of the cell culture media in the inlet and in the outlet of the reservoir, and to adapt the cell culture media and the flow rate continuously based on the combined information measured in the inlet and the outlet by the sensors permits to generate a homogeneous cell culture. Indeed, the 3D printed scaffold permits the generate an homogeneous flow of cell culture media inside the porosity of the scaffold by avoiding preferential path flow of cell culture media, while the presence of the inlet and outlet sensors at the inlet and the outlet of the reservoirs permits to measure the variation in the value of a cell culture parameter from the inlet to the outlet and to generate combined and comprehensive data. This allows a precise and continuous control, in real-time, of the conditions of cell culture inside the scaffold, increasing the homogeneity of the cell culture by avoiding detrimental gradient of O₂ and/or nutrients and/or others cell culture parameters from the inlet to the outlet of the scaffold. Moreover, the control element operatively connected to the actionable element in the recirculation loop permits to automatize the implementation of the bioreactor system allowing a simple implementation. This also permits an optimization of the adaptation of the cell culture media contributing to reduce the volume of cell culture media needed to implement the bioreactor system which renders the bioreactor system affordable in term of cost because of the reduced volume a cell culture media needed to reach an industrial production scale of cells and/or biomolecules of high and constant quality.
Figure 1 is a schematic representation of the bioreactor according to the invention.
Figure 2 is a schematic representation of a bioreactor of the invention comprising several reservoirs.

### Detailed description of the invention

The inventors have designed a compact bioreactor allowing the large-scale production of cell-derived material allowing an excellent control of culture parameters and minimizing gradients, which would have resulted into heterogeneities and thus into non-reliable end products.

A first aspect of the present invention is thus a bioreactor system 1 for culturing adherent cells, the system comprising:
- a cell culture vessel 2 comprising at least one interior reservoir 3, wherein the reservoir 3 comprises a porous scaffold 4 arranged to support the culture of said adherent cells,
- an inlet 5 fluidly connected to the reservoir 3 (at, or close to, one extremity of the reservoir), and an outlet 6 fluidly connected to the reservoir 3 (at, or close to, the other extremity of the reservoir), wherein the outlet 6 is (further to the connection through the porous 3D matrix) fluidly connected to the inlet 5 via a recirculation loop 11 of cell culture media, wherein the inlet 5 is (further) fluidly connected to a source of cell culture media 7,
- at least one inlet sensor 8a, 8b arranged in (e.g. at the inlet 5 of) the cell culture vessel 2, wherein the inlet sensor 8a, 8b is configured to measure a parameter of cell culture media entering the cell culture vessel 2 via the inlet 5,
- at least one outlet sensor 9 arranged at the outlet 6 of the cell culture vessel 2, wherein the outlet sensor 9 is configured to measure a parameter of cell culture media exiting the cell culture vessel 2 via the outlet 6,
- a pump 15, able to generate a flux of the cell culture media into the reservoir 3 and/or into the recirculation loop 11,
- a control element operatively connected to the inlet sensor(s) 8a, 8b, to the outlet sensor(s) 9, to the pump 15, and to an actionable element able to continuously modify said parameter of cell culture media, wherein the control element is arranged to precisely determine the value of the parameter of cell culture media measured at the outlet and at the inlet, and, based on said combined information, continuously adapt said parameter of cell culture media through a control of said actionable element, wherein the inlet and outlet sensors 8a, 8b, 9 comprise at least an oxygen sensor (e.g. to measure oxygen pressure) and wherein the porous scaffold 4 is a 3D printed scaffold.

The presence of the inlet and of the outlet sensors allows a precise monitoring of key parameters, at least oxygen; hence this allows to avoid important gradients of concentration (or of dissolved oxygen), which risks detrimental heterogeneities. The inventors have found that, at least for oxygen, it is important to have data upstream of the cell culture, to ensure these parameters are under acceptable ranges before entering the matrix, whereas the data downstream of the matrix allows to control potential heterogeneities (too important gradients through the matrix) and to adapt the system to reduce them (e.g. flux of the culture medium, concentration of dissolved oxygen in the vessel 2, outside the reservoir 3).

The recirculation of the culture medium allows savings, whereas the 3D printed porous matrix offers an important specific surface and also a homogenous circulation of the culture medium inside the matrix. Again, this is important to avoid heterogeneities, but also differences in shear stress applied to the cells in culture.

Preferably, the 3D printed porous matrix (i.e. the matrix forming the solid scaffold) comprises a plurality of channels allowing the cell culture medium flux to pass, from one extremity of the 3D printed matrix to the other extremity of the 3D printed matrix while ensuring a constant shear stress to the cells and/or a pressure kept between a minimal predetermined value and a maximal predetermined value. Advantageously, the 3D printed matrix is configured for avoiding preferential leakage in the lateral face of the said 3D printed matrix.

Preferably, the control element monitors in a continuous manner the variation between the parameter(s) of cell culture media measured at the outlet and at the inlet, and, based on such a combined information, adapt in a continuous manner said parameter(s) of cell culture media through a control of said actionable element.

Advantageously, the cell culture medium flux passing the outlet 6 or the outlet sensor(s) 9 is recycled back at the inlet 5 of the cell culture medium flux, preferably via the recirculation loop 11.

Preferably the internal diameter of the tubing in the circulation loop is larger than in other parts of the vessel 2 to avoid too large pressure while allowing a desired flux as well as allowing a pressure drop of maximum 0.1 bar (between the outlet 6 and the culture vessel 2. In addition, the diameter should be large enough to avoid too large linear speed of the medium, for instance a preferred linear speed within the 3D matrix 4 is kept below 30 cm/s.

On the other hand, the diameter should not be too large, to avoid increasing the proportion of the culture medium outside the 3D matrix 4.

Internal diameters from 0.3 to 1 cm are preferred.

Preferably, the length of the recirculation loop is comprised between - 10 cm and 150 cm, depending on the size and the purpose of the reactor (e.g. between 10 and 50 cm, preferably between 15 and 30 cm, or between 10 and 150 cm, preferably between 75 and 100 cm).

Preferably, the recirculation loop 11 is gas-impermeable.

Indeed, a potential entry of atmospheric oxygen would antagonize oxygen regulation, and affect culture conditions, which can be very detrimental if hypoxic conditions are needed. Furthermore, uncontrolled entry of atmospheric oxygen or release of carbon dioxide from the culture medium risks to cause an alkalinization of the culture medium.

Hence, silicone materials are not preferred. The inventors have used non-silicone Tygon^{®}-materials, yet several gas-impermeable materials can be used.

Preferably, the bioreactor 1 or the vessel 2 comprises an active mixing element 12 in fluid communication with external nutriments and/or with a tank comprising fresh culture medium, wherein the composition of the recycled cell culture medium flux, including the oxygen dissolved in the culture medium, is adapted continuously, as determined by the inlet and outlet sensors 8a, 8b, 9 and possible further based on pre-determined feeding strategies.

This ensures a culture medium with key parameters kept within acceptable ranges.

In addition, the gas (air, potentially supplemented with oxygen, nitrogen or carbon dioxide) is preferably supplied at the top of the bioreactor 1, above the level of the culture medium in the vessel 2. The mixing of gas streams (e.g. air + oxygen, and/or air + carbon dioxide, ...) allows an equilibrium between the added gas circulating above the culture medium 7 and the gas dissolved in the culture medium 7.

Hence, advantageously, the flux of the cell culture medium and/or the composition of the cell culture medium is determined continuously in function of
- the shear applied to the cells and of
- the values measured at the inlet and the outlet sensors 8a, 8b, 9.

Preferably, the actionable element of the system 1 is able to introduce into said interior reservoir:
- a regulated gas mix containing adaptable ration of air, oxygen, carbon dioxide and nitrogen-containing stream (introduced above the culture medium 7), and/or
- a continuously adaptable flux of cell culture medium, and/or
- a pH regulator.

Preferably, the 3D printed matrix 4 has a cylindric shape, the two extremities (inlet 5 and outlet 6) being the two circular bases of the said cylinder, preferably wherein the curved shape of the cylinder is surrounded by a water-tight membrane 13.

The water-tight (water-impermeable) and gas-tight membrane is preferably applied to the porous 3D printed matrix 4 by application of a retractable thermosetting water- and gas-tight membrane on the curved shape. This is especially easy to implement when the 3D printed matrix 4 has a cylindrical shape.

This ensures an absence of preferential lateral leakage of the cell culture medium and avoid the risk of introduction of sealing agent into the scaffold which can lead to heterogeneities in the outer sections of the matrix.

Moreover, the gas-impermeability ensures no introduction of gas into the matrix, risking also to create heterogeneities and perturb the cells conditions in the matrix (bubbles flowing in the matrix).

Advantageously, the scaffold (porous 3D printed matrix) 4 is defined by a volume bounded by
(i) an inlet face in fluid communication with the inlet,
(ii) an outlet face in fluid communication with the inlet,
(iii) at least one side face, wherein said side face is waterproof and gasproof.

As mentioned, the scaffold (porous 3D printed matrix) 4 is preferably surrounded by a waterproof and gasproof heat shrink tubing arranged to create a homogeneous flow throughout the scaffold 4 from the inlet 5 to the outlet 6.

Preferably, in operational mode, the volume of the cell culture medium present in the voids inside the scaffold (porous 3D printed matrix) 4 is comprised between 10% and 50%, preferably between 20% and 30%, of the total cell culture medium present in the voids inside the vessel 2 (hence including the scaffold 4) and in the recirculation loop 11.

Preferably, the inlet sensor 8a, 8b and outlet sensor 9 further comprise at least one additional sensor, selected from a pressure sensor, a pH sensor a sensor for lactate levels in the culture media, a flow meter, a carbon dioxide sensor, a thermometer, a cell-counter and a combination of several of these additional sensors.

The addition of carbon dioxide (or of carbon dioxide-enriched air) is advantageous especially for cells plated at low densities, for instance to avoid alkalinization of the medium.

Advantageously, the vessel 2 comprises a sensor for the minimal height of the culture medium and, preferably, one or more sensors for intermediate or maximal height of the medium. This ensures that the bottom of the 3D matrix 4 and the inlet 5 are immerged in the culture medium.

The inlet sensor 8a, 8b are preferably immerged in the culture medium and the top part of the vessel 2 is free of medium; hence filled with gas and able to receive a stream of gas having a composition determined by the controller and/or depending on the integrated data of the inlet and outlet sensors 8a, 8b, 9.

Indeed, since the pump 15 is preferably creating a suction force, the medium 7 does not need to fill the whole vessel 2 to allow filling the reservoir 3 and the 3D matrix 4.

The level of the culture medium in the vessel 2 can be adapted and can vary, depending on the needs.

More into details, this allows to increase, if/when needed, the level of the culture medium, depending on the needs.

Preferably, the outlet 6 allows to recover cells (for instance when several bioreactors 1 are arranged in series or in an assembly; see below), cell products of interest, such as biomolecules or extracellular vesicles of interest, and/or the outlet 6 allows to remove toxic metabolites produced by the cells in culture, or the exhaust cell culture medium. This is advantageously achieved with a specific valve 10, allowing either a recirculation into the recirculation loop 11, or the exit of medium from the outlet 6. Advantageously, the valve 10 further allows to work in counter-current, where an external medium or buffer is applied directly through the 3D printed matrix, without passing through the recirculation loop 11 or the vessel 2.

Alternatively, in counter-current, the inlet 5 can advantageously be configured to allow to recover cells or cell products.

Advantageously, the internal pressure is measured and/or adapted by an event 14, preferably wherein the event is equipped with an anti-droplet device, such as a system with internal protrusions from the top of the vessel 2, these protrusion being possibly a complete ring surrounding the entry of the event 14, or a plurality of spikes protruding downwards from the top of the vessel 2, or at least the distance (height) between the top of the vessel 2 (or the outlet 6) and the event 14 is sufficiently long to capture drops and allow them to fall back into the vessel 2. Preferably, the event 14 is a system where the opening to gas is controlled by the pressure.

The carbon dioxide produced by the cells (equilibrium between the cell medium at the outlet and the gas phase at the top of the vessel 2) and the flux of added gas pass advantageously through the event 14.

Preferably, the extremity of the recirculation loop 11 within the vessel 2, as well as any other feed inlets used for perfusion, is not too close to the event 14, and/or is immerged in the culture medium.

This avoids producing local droplets, at least droplets close to the event 14.

Indeed, such droplets of liquid risks to obturate the event 14

Preferably, the gas inlet(s) is/are equipped with a droplet-breaking device.

Another object of the present invention is an assembly of bioreactors 1, wherein each bioreactor 1 has different sizes and each bioreactor 1 is configured so that the cells are exposed to a constant shear stress and/or to a pressure kept between a predetermined lower value and a predetermined higher value, the said bioreactors 1 of different sizes being connected in series of increasing sizes.

This allows a system where culture conditions are kept constant, and to ensure the seeding of a sufficient cell concentrations, compatible with cell-cell-interactions.

In another related aspect, the bioreactor system comprises
- a series of interior reservoirs 3 arranged in parallel comprising each a scaffold 4 (a 3D printed matrix) having a volume and dedicated inlet and outlet sensors 8a, 8b, 9,
- at least a central pump 15 coupled to a common recirculation loop 11 arranged to transfer the cell culture media through the series of interior reservoirs 3.

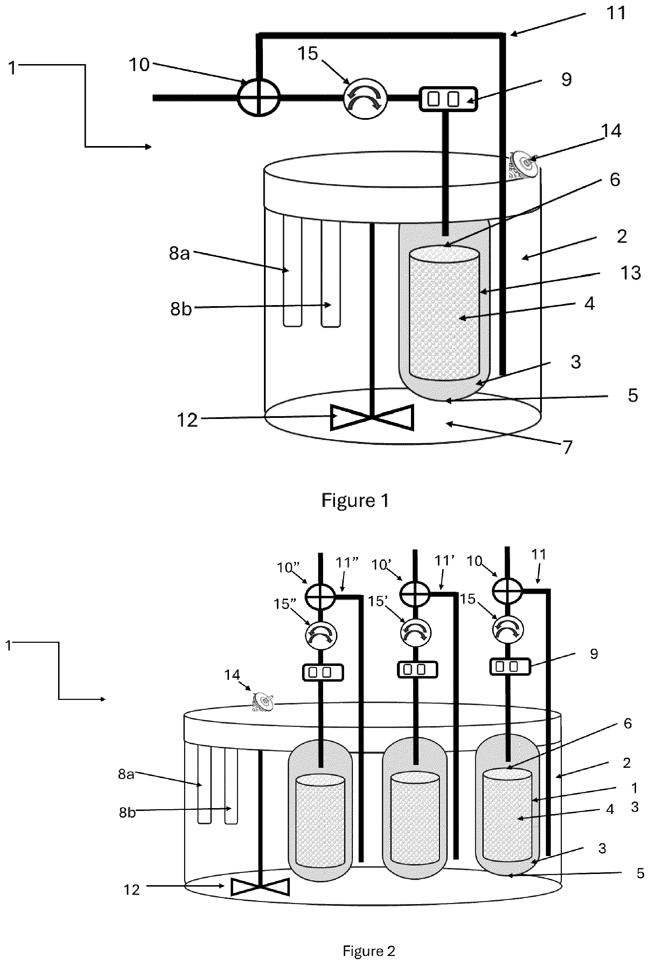

In another related aspect (Figure 2), the bioreactor system comprises
- a series of interior reservoirs 3 arranged in parallel comprising each a scaffold 4 (a 3D printed matrix) having a volume,
- separated recirculation loops, one per reservoir 3, and dedicated outlet sensors 9 per reservoir,
- inlet sensors 8a, 8b, and at least a central pump 15 arranged to transfer the cell culture media through the series of interior reservoirs 3.

It is to be noted that Figure 2 shows 3 reservoirs, yet this is not intended to be limiting as much larger number of reservoirs 3 can be inserted in parallel into one bioreactor system 1 and/or into one vessel 2.

These two systems in parallel allows mass-production, especially if it is not possible to rely on a too large 3D matrix (pressure, flow rate, shear, detrimental gradients within the matrix).

Another aspect of the present invention is a device for high-throughput screening comprising:
- a plurality of bioreactor systems 1 for culturing adherent cells (as described above),
- a central control unit operatively connected to each bioreactor system 1 of the plurality of bioreactor systems able to control each bioreactor system 1 individually.

This device allows to grow a plurality of cell cultures in exactly the same conditions, then to test different conditions; hence to precisely determine the effect of each condition, as controlled with the culture in another (i.e. a control) bioreactor system 1.

Another related aspect of the present invention is a process to grow adherent cells, comprising:
to seed the said adherent cells in the reservoir 3 described above or in the assembly of bioreactors 1 or in the device for high-throughput screening described above,
to continuously monitor the oxygen values upstream and downstream the 3D matrix 4,
to continuously adapt the stream of oxygen and the flux of cell culture medium in order to keep the values at a predetermined level and to keep the shear stress and the pressure applied to the cells between predetermined values.

Preferably, in this process, the flux of the culture medium within the 3D matrix 4 is determined in function of the shear stress and of the pressure acceptable by the cultured cells.

Advantageously, this process relies on an assembly comprising a plurality of identical bioreactors 1 or of identical vessels 2 or of identical reservoirs 3 further arranged in series and to
A) seed the adherent cells in a first bioreactor 1 or in the first vessel 2 or in the first reservoir 3 filled with the lowest amount of the culture medium,
B) grow the cells until a predetermined level of confluence,
C) detach the cells from the 3D matrix 4,
D) displace the detached cells in a second bioreactor 1 or in a second vessel 2 or in a second reservoir 3 of the assembly, being filled with a higher amount of the cell culture medium,
E) adhere the cells on the 3D matrix 4 of the said second reactor 1 or second vessel 2 or second reservoir 3,
F) grow the cells and,
optionally, to repeat (one or several times) the steps C to F in a subsequent bioreactor 1 or vessel 2 or reservoir 3 having an increased filling ratio by the culture medium.

This process is thus an alternative where the cell density and culture conditions are kept between acceptable ranges.

Possibly, in the other alternative, which is preferred, the culture process is based on the assembly described above (with different sizes of reservoirs 3) and comprises to
A) seed the adherent cells in the smallest bioreactor 1 or in the smallest vessel 2 or in the smallest reservoir 3,
B) grow the cells until a predetermined level of confluence,
C) detach the cells from the 3D matrix 4,
D) displace the detached cells in a second bioreactor 1 or in a second vessel 2 or in a second reservoir 3 of the assembly, being bigger than the first bioreactor 1 or the first vessel 2 or the first reservoir 3
E) adhere the cells on the 3D matrix 4 of the said second reactor 1 or the said second vessel 2 or the said second reservoir 3,
F) grow the cells and,
optionally, to repeat the steps C to F in a subsequent bioreactor 1 or vessel 2 or reservoir 3 having still an increased size.

Advantageously, there processes comprise a final step of harvesting the cells or the cell products, such as biomolecules produced by the cells or extracellular vesicles from the grown cells.

Preferably, this cell products are obtained after an induction step.

Preferably, the induction step is a variation of the pH, pressure, nitrogen concentration, shear stress, nutrient concentration, or the addition of an inducer chemical molecule.

In other words, the present invention relates to a method for culturing adherent cells by implementing the bioreactor system 1 described above or by implementing the device described above, the method comprising:
- a supply of said interior reservoir 2 containing said porous scaffold (3D printed matrix) 4 by a cell culture medium via said inlet 5, and, after a predefined duration, an exit via said outlet 6,
- a phase of seeding said porous scaffold (3D printed matrix) 4 with a starting number of cells resuspended in a volume of culture medium,
- a phase of attachment of said resuspended cells on said porous scaffold (3D printed matrix) 4,
- an expansion of the adherent cells on said porous scaffold (3D printed matrix) 4 up to a second number of adherent cells, said second number of adherent cells divided by said starting number defining a multiplication factor, wherein the multiplication factor is equal to at least 5 for a single expansion step in said scaffold (3D printed matrix)4.

Preferably, in this method, the multiplication factor is at least 10 during a single expansion step in said scaffold, preferably at least 15, even more preferably at least 40.

Preferably, these culture methods are further comprising:
- a production of extracellular vesicles by said adherent cells during a production phase, subsequent to or concomitantly with the expansion, during which the vesicles are produced and excreted in the culture medium to give a culture medium enriched with extracellular vesicles,
- a harvest of extracellular vesicles.

Another related aspect of the present invention is a method for high-throughput screening of a condition, comprising
to select a plurality of the bioreactor systems 1 described above,
to grow cells in the bioreactor systems 1
to screen different conditions of cell cultures wherein all but one of the culture conditions in each bioreactor system of the plurality of bioreactor systems are kept constant, and one condition of cell cultures differs as compared to the other bioreactor systems 1 of the plurality of bioreactor systems.

## Claims

1. A bioreactor system (1) for culturing adherent cells, the system comprising:
- a cell culture vessel (2) comprising at least one interior reservoir (3), wherein the reservoir (3) comprises a porous scaffold (4) arranged to support the culture of said adherent cells,
- an inlet (5) fluidly connected to the reservoir (3), and an outlet (6) fluidly connected to the reservoir (3), wherein the outlet is fluidly connected to the inlet via a recirculation loop (11) of cell culture media, wherein the inlet (5) is fluidly connected to a source of cell culture media (7),
- at least one inlet sensor (8a, 8b) arranged at the inlet of the cell culture vessel (2), wherein the inlet sensor (8a, 8b) is configured to measure a parameter of cell culture media entering the cell culture vessel (2) via the inlet (5),
- at least one outlet sensor (9) arranged at the outlet of the cell culture vessel (2), wherein the outlet sensor (9) is configured to measure a parameter of cell culture media exiting the cell culture vessel (2) via the outlet (6),
- a pump (15), able to generate a flux of the cell culture media into the reservoir (3) and/or into the recirculation loop (11),
- a control element operatively connected to the inlet sensor (8a, 8b), the outlet sensor (9), the pump (15), and to an actionable element able to continuously modify said parameter of cell culture media, wherein the control element is arranged to determine the variation between the parameter of cell culture media measured at the outlet and at the inlet, and, based on said combined values, continuously adapt said parameter of cell culture media through a control of said actionable element, wherein the inlet and outlet sensors (8a, 8b, 9) comprise at least an oxygen sensor and wherein the porous scaffold (4) is a 3D printed scaffold.

2. The bioreactor system according to claim 1, wherein the 3D printed porous matrix is a solid scaffold comprising a plurality of channels allowing the cell culture medium flux to pass, from one extremity of the 3D printed matrix (4) to the other extremity of the 3D printed matrix (4) while ensuring a constant shear stress to the cells and/or a pressure kept between a minimal predetermined value and a maximal predetermined value, the said 3D matrix (4) being configured for avoiding preferential leakage in the lateral face of the said 3D matrix (4).

3. The bioreactor system according to anyone of the preceding claims, wherein the cell culture medium flux passing the outlet sensor(s) (9) is recycled back at the inlet (5) of the cell culture medium flux, preferably wherein the tubing after the outlet sensor is impermeable to gas.

4. The bioreactor system according to claim 3, comprising an active mixing element (12) in fluid communication with external nutriments and/or with a tank comprising fresh culture medium, wherein the composition of the recycled cell culture medium flux is adapted continuously, as determined by the inlet and outlet sensors (8a, 8b, 9).

5. The bioreactor system according to anyone of the preceding claims, wherein the flux of the cell culture medium and/or the composition of the cell culture medium is determined continuously in function of
- the shear applied to the cells and of
- the values measured between the inlet and the outlet sensors (8a, 8b, 9).

6. The bioreactor system according to anyone of the preceding claims, wherein the 3D printed matrix (4) has a cylindric shape, the two extremities being the two circular bases of the said cylinder, preferably wherein the curved shape of the said cylinder is surrounded by a water-tight (water impermeable) and gas-tight membrane (13), preferably by application of a retractable thermosetting water-tight membrane on the said curved shape.

7. The bioreactor system according to anyone of the preceding claims, wherein, in operational mode, the volume of the cell culture medium present in the voids inside the scaffold (4) is comprised between 10% and 50%, preferably between 20% and 30%, of the total cell culture medium present in the voids inside the recirculation loop (11) and in the vessel (2).

8. The bioreactor system according to anyone of the preceding claims, wherein said at least one inlet sensor (8a, 8b) and said at least one outlet sensor (9) further comprises a pressure sensor, a pH sensor, a sensor for lactate levels in the culture media, a flow meter, cell-counter, a carbon dioxide sensor and/or a thermometer.

9. The bioreactor system according to anyone of the preceding claims, wherein the internal pressure is measured and adapted by an event (14), preferably wherein the event is equipped with an anti-droplet device.

10. The bioreactor system according to anyone of the preceding claims, comprising a gas inlet for delivering supply gas above the culture medium (7) present in the vessel (2), wherein the supplied gas is air, oxygen, carbon dioxide, nitrogen and mixtures thereof.

11. An assembly of bioreactors (1) according to any one of the preceding claims 1 to 10, wherein each bioreactor (1) has different sizes and each bioreactor (1) is configured so that the cells are exposed to a constant shear stress and/or to a pressure kept between a predetermined lower value and a predetermined higher value, the said bioreactors (1) of different sizes being connected in series of increasing sizes.

12. A device for high-throughput screening comprising:
- a plurality of bioreactor systems (1) for culturing adherent cells according to any one of the preceding claims 1 to 10,
- a central control unit operatively connected to each bioreactor system (1) of the plurality of bioreactor systems able to control each bioreactor system (1) individually.

13. A process to grow adherent cells, comprising to
- seed the said adherent cells in the reservoir (3) of the bioreactor system (1) according to any one of the preceding claims 1 to 10 or in the assembly of bioreactors (1) of claim 11 or in the device of claim 12,
- continuously monitor the oxygen values upstream and downstream the 3D matrix (4),
- continuously adapt the stream of oxygen and the flux of cell culture medium in order to keep the values at a predetermined level and to keep the shear stress and the pressure applied to the cells between predetermined values.

14. The process of claim 13 comprising to select the assembly of claim 11 further arranged in series and to
A) seed the adherent cells in a first bioreactor (1) or in the first vessel (2) or in the first reservoir (3) said first bioreactor (1) or in the first vessel (2) or in the first reservoir (3) being the smallest of the assembly,
B) grow the cells until a predetermined level of confluence,
C) detach the cells from the 3D matrix (4),
D) displace the detached cells in a second bioreactor (1) or in a second vessel (2) or in a second reservoir (3) of the assembly, having a bigger size,
E) adhere the cells on the 3D matrix (4) of the said second reactor (1) or second vessel (2) or second reservoir (3),
F) grow the cells and,
optionally, to repeat the steps C to F in a subsequent bioreactor (1) or vessel (2) or reservoir (3) having still a bigger size.

15. The process of claims 13 or 14 comprising a final step of harvesting the cells or cell products, such as biomolecules produced by the cells or extracellular vesicles from the grown cells, possibly preceded by an induction step, preferably through the valve (10).
